(19) Europäisches Patentamt European Patent Office Office européen des brevets

(11) **EP 4 371 416 A1**

(12) **EUROPEAN PATENT APPLICATION**

(43) Date of publication:
**22.05.2024 Bulletin 2024/21**

(21) Application number: **22383118.1**

(22) Date of filing: **18.11.2022**

(51) International Patent Classification (IPC):
*A23K 10/10* (2016.01)    *A23K 10/16* (2016.01)
*A23K 20/126* (2016.01)    *A23K 20/142* (2016.01)
*A23K 20/158* (2016.01)    *A23K 20/163* (2016.01)
*A23K 30/20* (2016.01)    *A23K 50/75* (2016.01)
*A23K 50/80* (2016.01)    *A23L 21/00* (2016.01)
*A23K 10/30* (2016.01)

(52) Cooperative Patent Classification (CPC):
**A23K 10/12; A23K 20/105; A23K 20/121;**
**A23K 20/126; A23K 20/163; A23K 50/75;**
**A23K 50/80; A23L 31/00; A23L 33/135;**
A23K 10/30

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Biozyme Inc.**
**St Joseph, MO 64504 (US)**

(72) Inventor: **IPHARRAGUERRE, Ignacio Rodolfo**
**08461 SANT ESTEVE DE PALAUTORDERA (ES)**

(74) Representative: **ZBM Patents - Zea, Barlocci &**
**Markvardsen**
**Rambla Catalunya, 123**
**08008 Barcelona (ES)**

(54) **ASPERGILLUS BIOMASS CONTAINING BIOACTIVE METABOLITES**

(57)    The present disclosure provides a process for preparing a composition comprising biomass from *Aspergillus* and bioactive compounds, the process comprising culturing *Aspergillus* in a liquid medium comprising an assimilable carbon source and inorganic nutrients, wherein the culturing comprises: (i) a fermentation under aerobic conditions followed by (ii) a fermentation under stress conditions comprising hypoxic stress and pH stress conditions. Further provided are compositions obtainable by this process and food or feed supplements containing said compositions.

EP 4 371 416 A1

## Description

### Technical Field

The present disclosure refers to the field of animal nutrition. In particular, the disclosure refers to a nutritional supplement obtained from fungus, more particular, the nutritional supplement comprises biomass from *Aspergillus* containing bioactive metabolites.

### Background Art

Growth performance and productivity of animals, in particular, consumable animals, has been traditionally enhanced by hormone and antibiotic therapy. However, as environmental awareness and safety concerns grow in relation to the meat industry (recently also fishery), these sectors have intensified the search for alternative strategies to optimize productivity.

One of these strategies is to supplement the standard diet with beneficial microorganisms, or products obtained from the fermentation of said microorganisms. In this sense, fungal biomass and fermentation products thereof have been found as a convenient source of food and feed supplements.

The beneficial effects of fungal biomass in animal nutrition have been attributed to its high nutritive value as well as to its content in bioactive metabolites (https://doi.org/10.3390/foods10112774). The fungal biomass derived through fermentation has been considered as a valuable source of bioactive compounds which provide health benefits. Previous studies have shown that supplementation with *Aspergillus* fermentation extracts improve bioaccessibility and the absorption of nutrients in the human digestive system. Other reports disclose an improvement of animal immune responses and oxidative stress (https://doi.org/10.3390/ani11061621).

*Aspergillus* biomass is conventionally obtained by culturing the filamentous fungus in a culture media, filtering the cultured fungus and drying. However, the general scheme is not optimal for bioactive compound recovery, many soluble bioactive substances may be lost during filtration and others are inactivated during conventional drying. Moreover, both the nature and yield of bioactive metabolites may vary depending on the culturing conditions.

There is therefore a need in the art to provide novel, affordable processes for producing *Aspergillus* biomass containing bioactive metabolites.

### Summary

The inventors have developed a new process for culturing *Aspergillus* that optimizes biomass yield as well as production of bioactive metabolites by the fungus. The process comprises a two-step fermentation process, the first step comprising aerobic conditions for biomass production and the second step comprising a fermentation under stress conditions. The product obtained by this new process has advantages for use as food or feed supplement. The new process is also beneficial in terms of overall efficiency and energy savings, which renders it more environmental-friendly than prior art methods.

A first aspect disclosed herein thus refers to a process for preparing a composition comprising biomass from a non-pathogenic *Aspergillus* and bioactive compounds, the process comprising culturing the non-pathogenic *Aspergillus* in a liquid medium comprising an assimilable carbon source and inorganic nutrients, wherein the culturing comprises: (i) a fermentation under aerobic conditions followed by (ii) a fermentation under stress conditions.

The process disclosed herein has other relevant advantages of industrial relevance. For example, it allows for a stringent control of the fermentation process, such that the yield and composition of the obtained biomass product is regulated. This is important for optimizing media use, reducing variability between batches, and assuring bioactivity of the product.

A second aspect disclosed herein refers to a composition comprising biomass from a non-pathogenic *Aspergillus* and bioactive compounds obtainable by the process as defined in the first aspect.

In addition to biomass from *Aspergillus,* which is highly nutritive on itself, the composition disclosed herein contains valuable bioactive compounds. Some of said bioactive compounds, such as enzymes, antioxidants, minerals, polyunsaturated fatty acids and fibers, are conventionally found in *Aspergillus* ferments. However, the particular process disclosed herein results in a composition which, in addition to these compounds, contains additional, less conventional, bioactive compounds that further enrich the product. As a result, the composition disclosed herein has surprising practical benefits when used as food or feed supplement. For example, as will be shown in the examples below, supplementation of feeds with the composition disclosed herein improves growth and resistance to viral infections (measured as reduced mortality) in shrimp, improves egg quality (more mass, better grading, stronger shell) and hen productivity (better feed efficiency, extended lifespan), and improves tolerance to heat stress in fruit flies.

A third aspect disclosed herein refers to a composition comprising biomass from a non-pathogenic *Aspergillus* and bioactive compounds, wherein the bioactive compounds are selected from the group consisting of heteroligosaccharides comprising galactose and mannose residues, mevalonolactone, isopropylmalic acid, and adonitol.

A fourth aspect refers to a food or feed supplement comprising a composition as defined in the second or third aspects.

A fifth aspect refers to a food or feed comprising a composition as defined in the second or third aspects.

Other aspects disclosed herein are related to use of the composition as defined in the second or third aspects for food or feed supplementation. For example, one aspect refers to a method for supplementing the diet of an animal, said method comprising adding to the diet a composition as defined in the second or third aspects. Another aspect refers to the composition as defined in the second or third aspects as a food or feed supplement. Another aspect refers to the composition as defined in the second or third aspects for use in supplementing the diet of an animal.

The composition disclosed herein, thanks to its functional properties, is considered a postbiotic. The product also has prebiotic activity. Thus, in another aspect, the disclosure refers to a composition as defined in the second or third aspects for use as a prebiotic or postbiotic.

**Brief Description of Drawings**

Fig. 1. Schematic representation of the process disclosed herein.

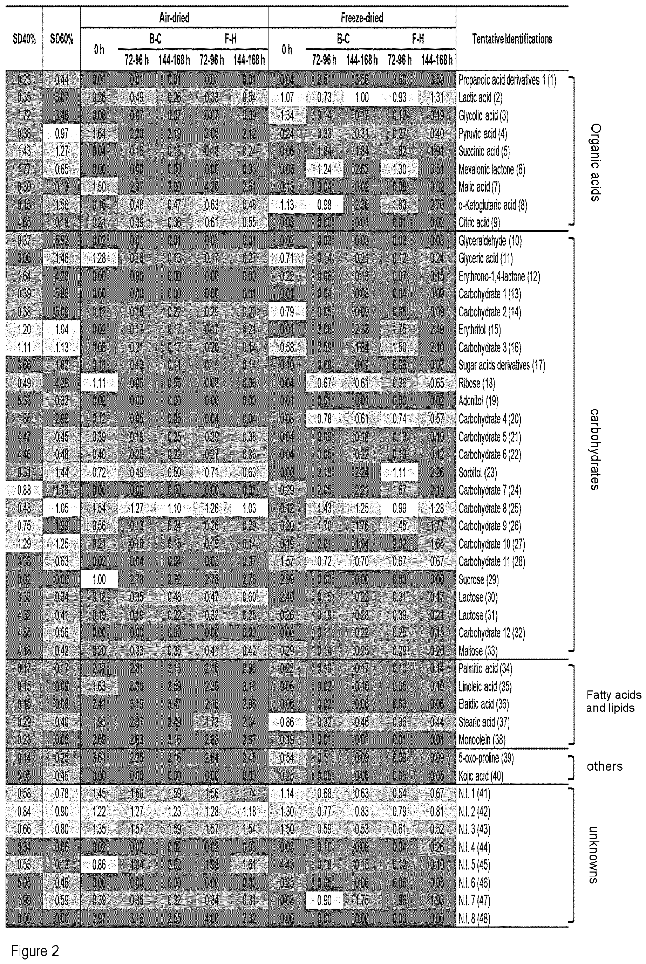

Fig. 2. Metabolite Heatmap for *Aspergillus oryzae* product obtained as detailed above dried on silicon dioxide at 40% wt/wt (AO40%); Amaferm® air dried on 50% wt/wt wheat bran (air dried) and lyophilized AO (freeze dried).

Fig. 3. Distribution of shrimp experimental diets in the RAS shelving system with three levels, showing the tank number and the allocated diet.

Fig. 4. Temperature change during cold stress event.

**Detailed description**

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

*The process*

The first aspect disclosed herein refers to a process for obtaining a composition comprising biomass from a non-pathogenic *Aspergillus* and bioactive compounds. The process comprises culturing *Aspergillus* by a two-step fermentation. A first fermentation (i) takes place under aerobic conditions and a second fermentation (ii) under stress conditions. The nutritional value and convenient bioactive metabolite profile renders this product useful as food or feed supplement.

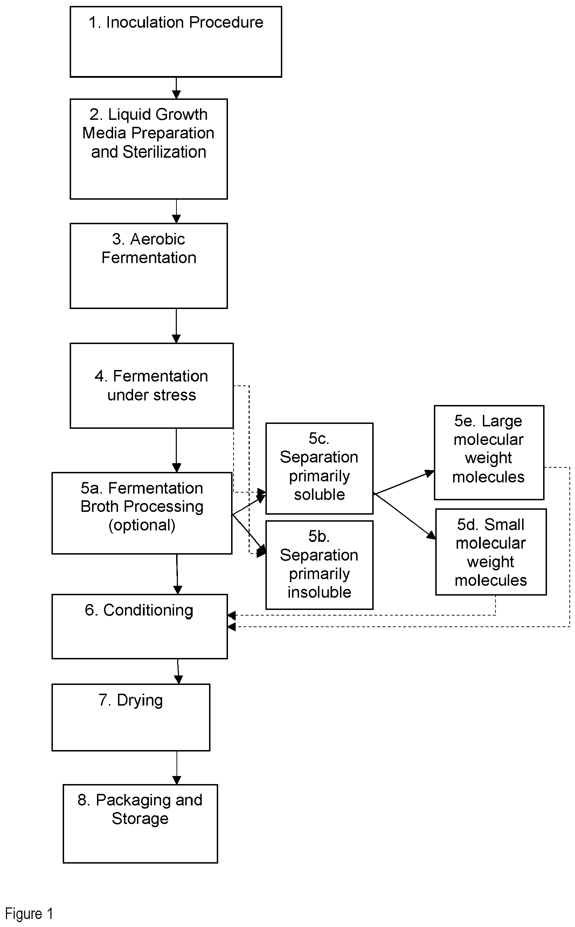

A schematic representation of the process is shown in figure 1.

*Fermentation*

The process starts by inoculating the fungus in a liquid medium. It has been found that strains of the *Aspergillus* group of fungi produce optimal amounts of bioactive compounds when cultured and treated according to the process disclosed herein. One of the strains which have proven very effective is *Aspergillus oryzae,* for example, *Aspergillus oryzae* NRRL 458, a culture of which is available as a deposit at the National Center for Agricultural Utilization Research in Peoria, Illinois. However, it has also been found that the production of an advantageous product by the process disclosed herein is not limited to any specific *Aspergillus,* and that certain strains of other fungi such as *Aspergillus* niger, *Aspergillus* wemii and *Aspergillus* tamarii are also appropriate. The strain of *Aspergillus,* for example, *Aspergillus oryzae,* for use in the sense of the present disclosure is non-pathogenic. In particular, the strain of *Aspergillus,* for example, *Aspergillus oryzae,* is GRAS (generally recognized as safe).

Culturing of *Aspergillus* may proceed in deep culture or liquid submerged culture. In one embodiment, fungal spores are inoculated in the fermentation medium. Germination of *Aspergillus* spores may be carried out by standard procedures in order to obtain enough spores for inoculation. This can be accomplished, for instance, by transferring an *Aspergillus* stock to a sterile germination culture medium, and incubating at appropriate conditions for growth and sporulation. *Aspergillus* spores are then harvested and used for inoculation into the fermentation medium. Appropriate conditions for growth and sporulation of the fungus may comprise 20-25 °C. The liquid medium for fermentation comprises an assimilable carbon source and inorganic nutrients. Various carbohydrates may be used as the assimilable carbon source in the culture medium. Sugars such as sucrose have been found to be an excellent carbon source, and these sugars need not necessarily be highly purified, but may be supplied from various sources, providing they are not contaminated by impurities which may interfere with the production of or render inactive the other constituents of the culture medium. Other possible sources of assimilable carbon are glucose, beet pulp, starch, maltose, maltodextrin (different chain lengths), glucose, fructose, galactose, lactose, sucrose, glycerol, mannitol, acetate, levoglucosan, mannose, arabinose, xylose, ribose, cellobiose, acetic acid, propionic acid, butyric acid, caproic acid, valeric, isovaleric and isobutyric acids. More than one source of assimilable carbon may be contained in the medium. The concentration of assimilable carbon source in the fermentation medium is typically from 15 to 50 g/L. For example, the fermentation medium may comprise from 20 to 40 g/L, for example, 21, 22, 23, 24, 25, 26, 27, 28, 29, 30, 31, 32, 33, 34, 35, 36, 37, 38, or 39 g/L of assimilable carbon source.

Besides one or more sources of assimilable carbon, the fermentation culture medium comprises additional nutrients which are required or are convenient for growth of the fungus. In particular, the fermentation culture medium also comprises inorganic nutrients, in particular, said inorganic nutrients are N and P. In addition to N and P, the fermentation culture may comprise at least one further inorganic nutrient selected from the group consisting of K, Mg, S, Fe, Zn, Co, Cu, Na, Mo, Ca, Cl, and combinations thereof. The fermentation culture may comprise one or more sources of the inorganic nutrients as defined above. This means that the fermentation culture comprises one or more sources of N and/or P, and optionally one or more sources of a further inorganic nutrient selected from the group consisting of K, Mg, S, Fe, Zn, Co, Cu, Na, Mo, Ca, Cl, and combinations thereof. These sources of inorganic nutrients are typically salts, for example selected from the group consisting of sodium nitrate, potassium phosphate dibasic, potassium phosphate monobasic, magnesium sulfate, potassium chloride, ferrous sulfate, ammonium nitrate, di-ammonium phosphate, ammonium phosphate, potassium nitrate, calcium chloride, zinc sulfate, copper sulfate, manganese sulfate, and combinations thereof. These salts can be used under various combinations and concentrations.

In some embodiments, the fermentation culture additionally comprises organic nutrients, such as, but not limited to, yeast extract.

In one embodiment, the amount of inorganic nutrients in the culture medium is from 0.001 to 20 g/L. In another embodiment, the amount of inorganic nutrients in the culture medium is from 0.01 to 10 g/L. In another embodiment, the amount of inorganic nutrients in the culture medium is from 0.001 to 0.1 g/L. In another embodiment, the amount of inorganic nutrients in the culture medium is from 1 to 10 g/L.

The fermentation culture medium may be adjusted to allow for the presence of mineral salts in the carbohydrate source, if a crude or impure source of carbohydrate is used. However, even if impure sources of nutrients may be used, it is generally convenient to use a fermentation medium of defined composition which allows for a stringent control of the fermentation process and guarantees absence of undesirable compounds in the final product.

The fermentation culture medium is typically adjusted to pH 5.8-6.2 before inoculating the fungal spores. As will be apparent to any skilled person, it is also desirable that the whole process takes place under measures to avoid contamination, which typically includes using sterile culture media and solutions.

The *Aspergillus* spores are inoculated into a culture medium as defined above for the first step of fermentation (aerobic fermentation). The inoculated medium is then cultured under aerobic conditions until the pH of the culture is in the range from 4.0 to 6.0, for example 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, or 5.9.. Other features marking the end of the first fermentation are, for example tertiary branching evident in the mycelia, glucose is <12.5g/L, and/or 50-100% nitrate consumption. Appropriate culture conditions for this first fermentation step may include a temperature of 28-35°C, typically 31-33 °C, aeration of 0.01-0.1VVM, and adequate mixing to provide for oxygen transfer and temperature control.

Appropriate aeration and mixing may be achieved, for example, by applying sterile supply of air coupled with an agitation system.

Upon completion of the first fermentation (i) as defined above, the culture is prepared for the second fermentation (ii), which takes place under stress conditions. For example, stress conditions comprise hypoxic stress conditions and pH stress conditions. "Hypoxic stress" is generally understood as reduced oxygen availability in the fermentation medium. It occurs during oxygen depletion due to growth of the *Aspergillus* in deep or submerged culture in the absence of aeration (or with insufficient aeration). "pH stress" is generally understood as non-optimal pH of the fermentation medium. In the sense of the present disclosure pH stress is particularly related to basic pH stress, i.e. a basic pH that is not optimal for fungal growth. Additionally, exposing the second fermentation to shear stress, such as that imparted by a centrifugal pump, may benefit the process.

Stress conditions may be conveniently achieved by increasing the pH of the culture and removing aeration. In particular embodiments the pH is increased above optimal pH for growth of the fungus, such as pH above 7, for example 7.5, 8, 8.5, 9, 9.5, 10, 10.5, 11, 11.5, 12 or 12.5. In other particular embodiments, the pH is increased to a lethal basic pH. In more particular embodiments the pH is increased to a close to lethal pH. The desired pH is typically achieved by adding a base, for example, calcium hydroxide.

Hypoxic stress is achieved by removing or significantly reducing aeration, such that oxygen is not transferred to the culture. By reducing aeration, the rate of oxygen diffusion is reduced which can be consider as an "stressful" intervention.

Next, the second the fermentation is allowed to proceed until the pH of the culture is in the range of 4-7.5, for example 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, or 7.4. Other features marking the end of first fermentation are, for example, lysis of tertiary mycelia of 50% of greater.

Fermentation may take place in any appropriate vessel. Industrial fermenters are available with the features required for carrying out the process disclosed herein. For example, the fermentation according to the present disclosure may be carried out in a fermenter capable of mixing with agitation and/or recirculation with a pump.

Preferably, by the end of the second fermentation the fungal culture is inactive. Inactivation of the fungus is relevant for some applications, for example, when the product is used as a postbiotic. The term "postbiotic" is generally recognized as a preparation of inanimate microorganisms and/or their components that confer a health benefit to the host. Inactivation of the culture may be ascertained by conventional methods, for example, by confirming the absence of viable *Aspergillus* colony formation under favorable culturing conditions. If required by the application, an active culture obtained by the second fermentation step may be inactivated by standard methods.

The fermentation broth resulting from the above process may be used directly or further processed. Further processing is desirable, for example, for reducing the water content or separating fractions of interest contained in the fermentation broth. In any case, it may be convenient to stabilize the ferment before further processing or direct consumption. Stabilization may be achieved by adding a composition comprising preservatives. For example, citric acid at 4.3g/L can be utilized for stabilization of the fermentation broth. Stabilization may also be achieved by adding a formulation comprising or consisting of Potassium sorbate, citric acid, calcium propionate, tocopherol, sorbic acid, Ethylenediaminetetraacetic acid, and ascorbic acid.

*Fractioning of the fermentation broth (optional)*

Optionally, following completion of the secondary fermentation (with or without stabilization), the fermentation broth may be separated into water soluble and water insoluble fractions. A shearing step to break down the particle size of the insoluble cell wall material can optionally be applied prior to separation. This can be achieved by means of centrifugation, filtration, screening, or similar technologies focused on size or density-based separations. The insoluble fraction, consisting of treated cell walls and precipitated compounds from secondary fermentation processing, can be utilized as a product directly, dried in the native form or conditioned with a carrier and dried (see below).

The soluble fraction can also be used as a product directly, dried in the native form, conditioned with a carrier and dried, or further processed. Many of the bioactive metabolites contained in the fermentation broth are contained in this soluble fraction. Further processing of this soluble fraction allows for fractioning or concentrating said bioactive metabolites. Thus, optionally, the soluble fraction of the broth can be further processed by means of filtration to separate small molecular weight components. This can be achieved by a process of ultrafiltration, nano-filtration, dialysis or reverse osmosis. With ultra and nano-filtration, the small molecular weight materials are passed through in the permeate of the filtration. With ultra and nano-filtration, the large molecular weight materials are concentrated in the retentate of the filtration.

Alternatively to, or in addition to, further processing of the soluble fraction from the fermentation broth, reverse osmosis can be applied directly to said soluble fraction to remove water at temperatures lower than that available with thermal methods of evaporation.

Each of the above fractions and subfractions can be mixed with carrier and dried separately or recombined in novel ratios to achieve the desired effect on the target species of animal through feed supplementation.

*Conditioning and drying*

The fermentation broth (or its fractions) obtained as described above, is generally further processed to reduce water content. In preferred embodiments enough water is removed to render a dry composition. Dry compositions are desirable for being more malleable, easier to handle and to transport. Dry compositions also have a lower water activity which allows for a longer stability and shell life. In particular embodiments, the whole fermentation broth is dried. Drying the whole fermentation broth has the advantage that no metabolites of interest are lost during intermediate manipulations such as filtering or centrifuging. In other embodiments, one or more fractions obtained from the fermentation broth are dried separately or combined in desired proportions and dried to achieve a particular effect.

However, drying the fermentation broth poses many practical and energetical challenges. The fermentation broth obtained by the above process typically contains about 97% water and 3% solids, which makes it very difficult to dry. Manipulation and energy consumption are of great concern when drying compositions with such a high water content.

The present inventors have found that most of the problems associated to drying a mixture of such high water content are solved by drying the fermentation broth by pulsed combustion drying. Thus, in one embodiment of the process disclosed herein, the fermentation broth is dried by pulsed combustion drying.

Pulsed combustion drying uses high-temperature pulsating jets generated by the pulse combustor to atomize and dry the liquid feed simultaneously. The term "pulse combustion" (PC) originates from the intermittent (pulse) combustion of the solid, liquid, or gaseous fuel in contrast to the continuous combustion in conventional burners. Such periodic combustion generates intensive pressure, velocity, and, to a certain extent, temperature waves propagated from the combustion chamber via a tailpipe to the process volume (applicator) such as a drying chamber. Because of the oscillatory nature of the momentum transfer, pulse combustion intensifies the rates of heat and mass transfer, thus accelerating drying rates.

Typically, pulse combustors oscillate with frequencies that vary from 20 to 200 Hz. Pressure oscillations in the combustion chamber of $\pm 10$ kPa produce tailpipe velocity oscillations of nominally $\pm 100$ m=s and the gas jet velocity at the tailpipe exit pulsates from approximately 0 to 100 m=s. The input power for commercially available pulse combustors ranges from 20 to 1000 kW.

Conditions in the dryer such as temperature, inert gas supplementation, pulse frequency, feed moisture content, and carrier type can be adjusted.

The drying conditions may be adjusted to remove the desired amount of water. The target moisture content after the drying may be less than 15% w/w, for example 14%, 13%, 12%, 11%, 10%, 9%, 8%, 7%, 6%, 5%, 4%, 3%, 2%, 1%, or 0.5%.

In addition to the energy savings and efficiency in water removal, it has been found that the PCD process highly affects the profile of bioactive compounds in the final composition. Compared with conventional drying methods, such as air drying or spray drying, PCD has been found to better maintain the bioactivity of the final composition by retaining the favorable metabolite profile. In contrast, conventional drying methods degrade some of the active metabolites, thus altering the bioactivity of the final composition.

In most embodiments, the fermentation broth (or its fractions) prepared as described above is conditioned by mixing with a carrier prior to drying. The skilled person may use any carrier that is appropriate according to the state of the art. In particular embodiments the carrier is an inorganic carrier, such as silicon dioxide, magnesium sulfate, calcium carbonate, calcium citrate, calcium phosphate, sodium chloride, potassium chloride, clay, dextrins, dextrose, maltodrextrins, bran, or combinations thereof. In other embodiments the carrier is an organic carrier, such as dextrins, dextrose, maltodrextrins, or bran. The weight ratio of ferment to carrier is typically within the range of 6:94 to 70:30. In particular embodiments the weight ratio ferment:carrier is within the range of 10:90 to 60:40, or from 20:80 to 50:50, or from 30:70 to 40:60, for example 35:65. The inventors have found that conditioning with $SiO_2$ is advantageous. For example, the ferment may be mixed with silicon dioxide at a weight ratio ferment:$SiO_2$ within the ranges mentioned above. In a particular embodiment, the fermentation broth is mixed with $SiO_2$ at a ferment:$SiO_2$ weight ratio from 30:70 to 40:60, for example 35:65. When insoluble carriers are used, such as silicon dioxide, mixing of the ferment and the carrier may be achieved by briefly mixing with a high shear mixer to fully incorporate the 2 feeds and then mixing with lower shear to prevent particle size degradation. The mixture is then submitted to drying. Oxidation may be avoided through the mixing process by low residence time in mixing tanks with proper agitation and addition of anti-oxidants prior to the drying.

This conditioning step also affects the bioactive metabolite profile of the final composition.

*The composition*

The second aspect disclosed herein refers to a composition obtainable, or obtained, by the process defined above. Said composition comprises biomass from *Aspergillus,* for example and bioactive compounds produced by the fungus during fermentation. The third aspect disclosed herein further provides for a composition comprising biomass from *Aspergillus* and bioactive metabolites.

The biomass in the composition of the second or third aspects may be from any non-pathogenic *Aspergillus,* such as *Aspergillus oryzae* and, in particular, from *Aspergillus oryzae* strains, for example, *Aspergillus oryzae* NRRL 458, or indeed other *Aspergillus* species, for example, selected from *Aspergillus* niger, *Aspergillus* wemii and *Aspergillus* tamarii.

The bioactive metabolites in the compositions are one or more of those disclosed in the metabolite profile shown in the examples below. Generally speaking, the bioactive metabolites in the composition of the second or third aspects disclosed herein are selected from the group consisting of heteroligosaccharides comprising galactose and mannose residues, mevalonolactone, isopropylmalic acid, and adonitol. The composition of the second or third aspects may further comprise a metabolite selected from the group consisting of succinic acid, sebacic acid, glycolic acid, and eyrthrono-1,4 lactone, glyceric acid, kojic acid, sebacic acid, isopropylmalic acid, lysophosphatidylethanolamine 18:2, lysophosphatidylcholine 18:1, 9,10,13 TriHOME, 9,12,13 TriHOME, 12,13 DiHOME, azelaic acid, and combinations thereof. For example, the composition of the second or third aspects may comprise heteroligosaccharides comprising galactose and mannose residues, mevalonolactone, isopropylmalic acid, adonitol, succinic acid, sebacic acid, glycolic acid, and eyrthrono-1,4 lactone. In another example, the composition comprises all of the above metabolites. In another example, it may comprise heteroligosaccharides comprising galactose and mannose residues, mevalonolactone, isopropylmalic acid, adonitol, and one or more further metabolites selected from those disclosed in Table 1. In particular, the heteroligosaccharides comprising galactose and mannose residues are alpha-linked heteroligosaccharides.

The amount of bioactive compounds in the composition may vary depending on several factors, for example, it may depend from the extent of water removal or from the conditioning process. For example, the composition of the second or third aspects disclosed herein may comprise from 0.01 to 10% (w/w) of bioactive metabolites, such as those mentioned above. In particular, the amount of the bioactive compounds as defined above in the compositions may be from 0.1 to 5 % (w/w), for example from 0.5 to 3%, such as 0.5%, 0.8%, 1%, 1.2%, 1.5%, 1.8%, 2%, 2.3%, 2.5%, or 2.7%. The expression "w/w" is understood as the weight percentage of the referred compound(s) with respect to the total weight of the final composition in other words, x % (w/w) of compound A represents x grams of ingredient A in 100 g of the total composition.

The compositions disclosed herein preferably have a low water content. For example, the water content of the compositions of the second or third aspects disclosed herein may be less than 15% w/w, or less than 10% w/w, or less than 7% (w/w), or less than 5% w/w, or less than 4%, or even less than 3%.

*Uses of the composition*

The compositions disclosed herein are highly nutritive and confer additional health benefits on the host. For example, the compositions disclosed herein may be for use in improving bioavailability and/or the absorption of nutrients in the host's digestive system. Further, the compositions disclosed herein may be for use in improving growth, productivity and/or resistance to viral infections in host animals. The compositions disclosed herein may also be for use in improving resistance to stress condition in host animals. As such, the compositions disclosed herein are useful for animal nutrition, in particular for animal food or feed supplementation.

The present disclosure thus provides food or feed supplements comprising the compositions disclosed herein. The food or feed supplement may essentially comprise the composition disclosed herein, optionally with small amounts of food or feed additives. Alternatively, the food or feed supplement may comprise other ingredients, such as carries and/or other active ingredients.

Moreover, the disclosure provides for a food or feed comprising a composition as defined above. Also provided is a food or feed comprising a food or feed supplement as defined above. The food or feed may typically comprise a small amount of the composition or the supplement disclosed herein . For example, the food or feed may comprise from 0.001 to 5% (w/w) of the composition disclosed herein. In particular embodiments the food or feed may comprise from 0.001 to 2% (w/w) of the composition disclosed herein. More particularly the food or feed may comprise from 0.001 to 10% (w/w), or from 0.001 to 1% (w/w), or from 0.001 to 0.1% (w/w), or from 0.002 to 0.1 % (w/w) or from 0.003 to 0.1% (w/w), or from 0.004 to 0.1% (w/w) of the composition disclosed herein, or from 0.005 to 0.1% (w/w) of the composition disclosed herein. For example the food or feed may comprise 0.005%, 0.01%, 0.025%, 0.05%, 0.1%, or 1% of the composition disclosed herein.

The food or feed may comprise, in addition to a certain amount of the composition disclosed herein, additional food or feed components. For example, the additional food or feed component may be selected from protein sources, carbohydrate sources, roughage, silage, fats, vitamins, minerals and trace elements.

For completeness, the present description also disclosed the following numbered embodiments:

1. A process for preparing a composition comprising biomass from a non-pathogenic *Aspergillus* and bioactive compounds, the process comprising culturing the non-pathogenic *Aspergillus* in a liquid medium comprising an assimilable carbon source and inorganic nutrients.

2. The process according to the previous embodiment, wherein the culturing comprises fermentation in deep cultue.

3. The process according to the embodiment 1, wherein the culturing comprises liquid submerged fermentation.

4. The process according to any one of the previous embodiments, wherein the culturing comprises: (i) a fermentation under aerobic conditions followed by (ii) a fermentation under stress conditions.

5. The process according to the preceding embodiment, wherein stress conditions comprise hypoxic stress.

6. The process according embodiment 4, wherein stress conditions comprise pH stress

7. The process according to the preceding embodiment, wherein pH stress conditions comprise a sub-lethal basic pH.

8. The process according to the preceding embodiment, wherein pH stress conditions comprise culturing the non-pathogenic *Aspergillus* in a medium having an initial pH in a range from 8 to 12.

9. The process according to any one of the preceding embodiments, wherein the carbon source in the liquid medium is selected from the group consisting of sucrose, glucose, beet pulp, starch, maltose, maltodextrin, glucose, fructose, galactose, lactose, and combinations thereof.

10. The process according to any one of the preceding embodiments, wherein the assimilable carbon source is present in the liquid medium is present in an amount from 15 to 50 g/L.

11. The process according to the preceding embodiment, wherein the assimilable carbon source is present in the liquid medium is present in an amount from 20 to 40 g/L.

12. The process according to any one of the preceding embodiments, wherein the liquid medium comprises inorganic nutrients selected from the group consisting of K, Mg, S, Fe, Zn, Co, Cu, Na, Mo, Ca, Cl, and combinations thereof.

13. The process according to the preceding embodiment, wherein the liquid medium comprises salts of K, Mg, S, Fe, Zn, Co, Cu, Na, Mo, Ca, Cl, and combinations thereof.

14. The process according to embodiment 12, wherein the liquid medium comprises N and P.

15. The process according to embodiment 13, wherein the liquid medium comprises salts of N and P.

16. The process according to any one of embodiments 12-15, wherein the liquid medium comprises sodium nitrate, potassium phosphate dibasic, potassium phosphate monobasic, magnesium sulfate, potassium chloride, ferrous sulfate, ammonium nitrate, di-ammonium phosphate, ammonium phosphate, potassium nitrate, calcium chloride, zinc sulfate, copper sulfate, manganese sulfate, and combinations thereof.

17. The process according to any one of embodiments 12-16, wherein the amount of inorganic nutrients in the culture medium is from 0.001 to 20 g/L.

18. The process according to the preceding embodiment, wherein the amount of inorganic nutrients in the culture medium is from 0.01 to 10 g/L.

19. The process according to embodiment 17, wherein the amount of inorganic nutrients in the culture medium is from 0.001 to 0.1 g/L.

20. The process according to embodiment 17, wherein the amount of inorganic nutrients in the culture medium is from 1 to 10 g/L.

21. The process according to any one of the preceding embodiments, wherein:

- the fermentation under aerobic conditions (i) is allowed to proceed until the pH of the culture is in the range from 4.0 to 6.0,
- the culture is adjusted to pH from 8 to 12 before the fermentation under stress conditions (ii), and

the fermentation under stress conditions (ii) is allowed to proceed until the pH of the culture is in the range from 4.0 to 7.5.

22. The process according to the preceding embodiment, wherein the fermentation under aerobic conditions (i) is allowed to proceed until tertiary branching is evident in the mycelia.

23. The process according to any one of embodiments 21-22, wherein the culture is adjusted to sub-lethal pH or close to thai pH before the fermentation under stress conditions (ii)

24. The process according to any one of embodiments 21-23, wherein the fermentation under stress conditions (ii) is allowed to proceed until the pH of the culture is in the range from 4.0 to 6.0.

25. The process according to any one of the preceding embodiments, wherein upon completion of the fermentation the *Aspergillus* is inactive.

26. The process according to any one of the preceding embodiments, further comprising stabilizing the composition, for example, by adding citric acid.

27. The process according to any one of the preceding embodiments, further comprising fractioning the resulting fermentation broth.

28. The process according to any one of the preceding embodiments, further comprising mixing the resulting fermentation broth with a carrier material.

29. The process according to the preceding embodiment, wherein the carrier is selected from the group consisting of silicon dioxide, magnesium sulfate, calcium carbonate, calcium citrate, calcium phosphate, sodium chloride, potassium chloride, clays, dextrins, dextrose, maltodrextrins, bran, and combinations thereof.

30. The process according to the preceding embodiment, wherein the carrier is silicon dioxide.

31. The process according to any of the embodiments 29-30, wherein the composition and the carrier material are mixed at a weight ratio ranging from 6:94 to 70:30.

32. The process according to the preceding embodiment, wherein the composition and the carrier material are mixed at a weight ratio ranging from 15:85 to 35:65.

33. The process according to any one of the preceding embodiments 16-19, further comprising a step of drying.

34. The process according to the preceding embodiment, wherein the drying is performed by pulsed combustion drying.

35. The process according to any one of embodiments 33-34, wherein the moisture content after drying is less than 15% w/w.

36. The process according to the preceding embodiment, wherein the moisture content after drying is less than 5% w/w.

37. The process according to anyone of the preceding embodiments, wherein the bioactive compounds in the composition are selected from the group consisting of heteroligosaccharides comprising galactose and mannose residues, succinic acid, mevalonolactone, sebacic acid, isopropylmalic acid, adonitol, glycolic acid, eyrthrono-1,4 lactone, and combinations thereof.

38. The process according to the preceding embodiment, wherein the composition comprises heteroligosaccharides comprising galactose and mannose residues, succinic acid, mevalonolactone, sebacic acid, Isopropylmalic acid, adonitol, glycolic acid, and eyrthrono-1,4 lactone.

39. The process according to any one of embodiments 37-38, wherein the composition further comprises a compound selected from the group consisting of glyceric acid, kojic acid, sebacic acid, isopropylmalic acid, lysophosphatidylethanolamine 18:2, lysophosphatidylcholine 18:1, 9,10,13 TriHOME, 9,12,13 TriHOME, 12,13 DiHOME, azelaic acid, and combinations thereof.

40. The process according to any of embodiments 37-39, wherein the composition further comprises glyceric acid, kojic acid, sebacic acid, isopropylmalic acid, lysophosphatidylethanolamine 18:2, lysophosphatidylcholine 18:1, 9,10,13 TriHOME, 9,12,13 TriHOME, 12,13 DiHOME, and azelaic acid.

41. The process according to any of the preceding embodiments, wherein the amount of the bioactive compounds in the composition is from 0.01 to 10% (w/w).

42. The process according to the preceding embodiment, wherein the amount of the bioactive compounds in the composition is from 0.1 to 5% (w/w).

43. The process according to any of the preceding embodiments, wherein the *Aspergillus* is a non pathogenic *Aspergillus.*

44. The process according to the preceding embodiment, wherein the *Aspergillus* is *Aspergillus oryzae.*

45. The process according to the preceding embodiment, wherein the *Aspergillus* is *Aspergillus oryzae* NRRL 458.

46. A composition comprising biomass from *Aspergillus* and bioactive compounds obtainable by the process as defined in any one of the preceding embodiments.

47. A composition comprising biomass from *Aspergillus* and bioactive compounds.

48. The composition according to the preceding embodiment, wherein the bioactive compounds in the composition include heteroligosaccharides comprising galactose and mannose residues.

49. The composition according to any one of embodiments 47-48, wherein the bioactive compounds in the composition include succinic acid.

50. The composition according to any one of embodiments 47-49, wherein the bioactive compounds in the composition include mevalonolactone.

51. The composition according to any one of embodiments 47-50, wherein the bioactive compounds in the composition include sebacic acid.

52. The composition according to any one of embodiments 47-51, wherein the bioactive compounds in the composition include isopropylmalic acid.

53. The composition according to any one of embodiments 47-52, wherein the bioactive compounds in the composition include adonitol.

54. The composition according to any one of embodiments 47-53, wherein the bioactive compounds in the composition include glycolic acid.

55. The composition according to any one of embodiments 47-54, wherein the bioactive compounds in the composition include eyrthrono-1,4 lactone.

56. The composition according to any one of embodiments 47-55, further comprising a compound selected from the group consisting of glyceric acid, kojic acid, sebacic acid, isopropylmalic acid, lysophosphatidylethanolamine 18:2, lysophosphatidylcholine 18:1, 9,10,13 TriHOME, 9,12,13 TriHOME, 12,13 DiHOME, azelaic acid, and combinations thereof.

57. The composition according to any one of embodiments 47-56, further comprising glyceric acid, kojic acid, sebacic acid, isopropylmalic acid, lysophosphatidylethanolamine 18:2, lysophosphatidylcholine 18:1, 9,10,13 TriHOME, 9,12,13 TriHOME, 12,13 DiHOME, and azelaic acid.

58. The composition according to any one of embodiments 47-57, wherein the amount of the bioactive compounds in the composition is from 0.01 to 10% (w/w).

59. The composition according to the preceding embodiment, wherein the amount of the bioactive compounds in the composition is from 0.1 to 5% (w/w).

60. The composition according to any one of the embodiments 47-59, wherein the moisture content is less than 5% w/w.

61. The composition according to any one of the embodiments 47-60, wherein the *Aspergillus* is a non-pathogenic *Aspergillus.*

62. The composition according to the preceding embodiment, wherein the *Aspergillus* is *Aspergillus oryzae.*

63. The composition according to the preceding embodiment, wherein the *Aspergillus* is *Aspergillus oryzae* NRRL 458.

64. A food or feed supplement comprising a composition as defined in any one of embodiments 46-63.

65. A food or feed comprising a composition as defined in any one of embodiments 46-63 or comprising a supplement as defined in embodiment 64.

66. A food or feed according to the preceding embodiment, further comprising at least one food or feed component selected from protein sources, carbohydrate sources, roughage, silage, fats, vitamins, minerals and trace elements.

67. The composition according to any one of embodiments 46-63, wherein said composition is a postbiotic.

68. The composition according to any one of embodiments 46-63, wherein said composition is a prebiotic.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the disclosure. The following examples and drawings are provided by way of illustration, and they are not intended to be limiting of the present disclosure. Furthermore, the present disclosure covers all possible combinations of particular and preferred embodiments described herein.

Examples

Example 1. Preparation of *Aspergillus* **oryzae** biomass composition

A schematic representation of the process is shown in figure 1. Fermentations took place in a sterile production fermenter.
[1,2] Rehydrated spores of *Aspergillus oryzae* NRRL 458 were inoculated into sterile culture medium containing 30g/L sucrose and inorganic nutrients (sodium nitrate and potassium phosphate dibasic magnesium sulfate, potassium chloride, and ferrous sulfate) at a pH of 5.8-6.2. [3] Fermentation proceeded at temperature of 31-33C, aeration, and adequate mixing to provide for oxygen transfer and temperature control. The primary aerobic fermentation was allowed to proceed until quaternary branching was evident in the mycelia.
[4] Upon completion of the primary ferment, aeration was removed, calcium hydroxide was added to achieve a pH of 9.5-11.5, the culture was allowed to continue until the pH dropped to 5-6. [6] The ferment was then conditioned for drying by mixing with silicon dioxide. The ratio of ferment to carrier was such that the dry solids ratio of ferment:carrier was around 40:60. [7] This mixture was then pumped to a pulse combustion dryer with an outlet temperature setpoint of 170-200F and a target moisture content of less than 5% w/w. Selective conditions in the dryer included: temperature, inert gas supplementation, pulse frequency, feed moisture content, and carrier type
The metabolite profile of the product obtained by the above method was determined by gas chromatography coupled to time-of-flight mass spectrometry (GC-TOF-MS). The results are shown on Table 1:

| No | Ret (min) | VIP1 | VIP2 | Metabolites | Unique Mass (*m/z*) | MS Fragment pattern (m/z) | ID |
|---|---|---|---|---|---|---|---|
| *Organic acids* | | | | | | | |
| 1 | 4,91 | 0,92 | 1,21 | Propanoic acid derivatives 1 | 174 | 73, 59, 174, 89, 58, 115, 75, 72 | MS |
| 2 | 5,03 | 1,69 | 1,32 | Lactic acid | 117 | 73,147,117,75,66,59,148,191 | STD/MS |
| 3 | 5,18 | 1,38 | 1,27 | Glycolic acid | 66 | 73, 147, 66, 59, 75, 148, 177, 133 | MS |

(continued)

| No | Ret (min) | VIP1 | VIP2 | Metabolites | Unique Mass (*m/z*) | MS Fragment pattern (m/z) | ID |
|---|---|---|---|---|---|---|---|
| *Organic acids* | | | | | | | |
| 4 | 5,90 | 1,39 | 1,28 | Pyruvic acid | 147 | 73, 147, 59, 75, 133, 72, 100, 148 | STD/MS |
| 5 | 7,62 | 0,35 | 1,16 | Succinic acid | 247 | 147,73,148,55,247,129,172,149 | STD/MS |
| 66 | 7,88 | 1,28 | 1,26 | Fumaric acid | 245 | 73, 147, 75, 245, 99, 83, 143, 133, 53 | STD/MS |
| 6 | 8,17 | 1,35 | 1,22 | Mevalonolactone | 145 | 73,115,145,194,92,65,150,143 | MS |
| 8 | 9,89 | 1,77 | 1,32 | α-Ketoglutaric acid | 198 | 73, 55, 147, 59, 74, 89, 198, 156, 112 | STD/MS |
| *Carbohydrates* | | | | | | | |
| 10 | 7,03 | 1,50 | 1,29 | Glyceraldehyde | 70 | 73, 147, 70, 103, 59, 89, 84, 75, 133, 117 | MS |
| 11 | 7,79 | 1,35 | 1,18 | Glyceric acid | 189 | 73, 147, 189, 133, 103, 75, 59, 117 | STD/MS |
| 14 | 9,13 | 1,50 | 1,29 | Carbohydrate 2 | 103 | 73, 103, 89, 59, 147, 133, 117, 173 | MS |
| 15 | 9,38 | 0,41 | 1,16 | Erythritol | 217 | 73, 147, 103, 117, 217, 205, 133, 116 | STD/MS |
| 17 | 10,40 | 1,23 | 1,23 | Sugar acids derivatives | 117 | 73, 117, 147, 59, 133, 103, 130,217 | MS |
| 72 | 10,54 | 1,09 | 1,12 | Carbohydrates 19 | 103 | 73, 117, 103, 147, 59, 101, 217, 133 | MS |
| 18 | 10,76 | 1,47 | 1,29 | Ribose | 217 | 73, 103, 147, 117, 89, 59, 205, 217, 133 | STD/MS |
| 50 | 11,07 | 1,10 | 1,21 | Carbohydrate 13 | 217 | 73, 103, 147, 217, 129, 117, 205, 133 | MS |
| 19 | 11,21 | 1,67 | 1,30 | Adonitol | 359 | 73, 147, 103, 359, 133, 117,217, 129 | STD/MS |
| 20 | 11,32 | 0,68 | 1,20 | Carbohydrate 4 | 231 | 73, 231, 142, 147, 58, 103, 133, 117 | MS |
| 22 | 11,59 | 1,58 | 1,28 | Carbohydrate 6 | 103 | 73, 217, 103, 147, 89, 133, 59, 257, 129 | MS |
| 68 | 12,03 | 1,47 | 1,29 | Fructose | 103 | 73, 103, 147, 160,217, 133, 117, 129 | STD/MS |
| 24 | 12,69 | 1,01 | 1,11 | Carbohydrate 7 | 260 | 73, 103, 147, 217, 89, 117, 133, 129 | MS |
| 25 | 12,87 | 1,05 | 1,19 | Carbohydrate 8 | 169 | 73, 103, 147, 217, 129, 204, 133, 205 | MS |
| 26 | 12,92 | 1,12 | 1,15 | Carbohydrate 9 | 204 | 73, 204, 103, 147,217, 129, 191,205 | MS |
| 27 | 13,20 | 0,25 | 1,16 | Carbohydrate 10 | 204 | 73, 204, 147, 103, 217, 205, 129, 133 | MS |
| 28 | 15,48 | 1,59 | 1,29 | Carbohydrate 11 | 173 | 73, 103, 173, 147,217, 117, 129, 133 | MS |
| 30 | 16,93 | 1,68 | 1,26 | Lactose | 217 | 73, 103, 217, 147, 129, 133, 117, 169 | STD/MS |
| 31 | 17,01 | 1,68 | 1,30 | Lactose | 217 | 73, 103, 217, 147, 129, 117, 133, 361 | STD/MS |
| 32 | 17,11 | 0,00 | 1,17 | Carbohydrate 12 | 361 | 73, 103, 217, 147, 129, 169, 361, 117 | MS |
| 33 | 17,18 | 1,64 | 1,30 | Maltose | 160 | 73,217, 103, 129, 117, 133, 160, 361 | STD/MS |
| *Fatty acids and lipids* | | | | | | | |
| 74 | 8,02 | 0,10 | 1,12 | Pentanoic acid deriv. | 240 | 73, 147, 240, 59, 166, 117, 85, 115 | MS |
| 36 | 14,19 | 1,03 | 0,87 | Elaidic acid | 339 | 73, 117, 129, 67, 81, 69, 145, 54, 339 | STD/MS |
| 37 | 14,32 | 0,68 | 1,15 | Stearic acid | 117 | 73, 117, 129, 132, 131, 100, 145, 341 | STD/MS |

(continued)

| **Fatty acids and lipids** | | | | | | | |
|---|---|---|---|---|---|---|---|
| 57 | 15,31 | 0,13 | 1,16 | Oleamide | 144 | 73, 131, 116, 144, 128, 67, 81, 132, 338 | STD/MS |
| **Amino acids** | | | | | | | |
| 39 | 9,50 | 0,72 | 1,17 | 5-oxo-proline | 156 | 73,156,147,103,59,117,133,173 | STD/MS |
| 58 | 9,52 | 0,14 | 1,10 | Aspartic acid | 232 | 73, 232, 147, 70, 59, 144, 233, 133 | STD/MS |
| **Others** | | | | | | | |
| 59 | 4,80 | 1,28 | 1,24 | 2,3-butanediol | 117 | 73,117,147,75,59,66,133,148 | MS |
| 60 | 7,26 | 0,22 | 1,18 | Adenosine-5'-diphosphate | 299 | 73, 299, 133, 211, 300, 135, 193 | STD/MS |
| 40 | 10,67 | 1,68 | 1,30 | Kojic acid | 271 | 73, 147, 271, 59, 69, 153, 197 | STD/MS |
| **Unknowns** | | | | | | | |
| 41 | 4,13 | 0,88 | 1,14 | N.I. 1 | 184 | 77, 73, 134, 69, 184, 58, 78, 75, 60 | - |
| 61 | 4,28 | 0,83 | 1,17 | N.I. 9 | 103 | 73, 103, 117, 58, 61, 59, 74, 55, 93 | - |
| 43 | 7,54 | 0,55 | 1,16 | N.I. 3 | 107 | 73, 107, 77, 69, 55, 84, 140, 59, 118 | - |
| 62 | 7,72 | 0,12 | 1,17 | N.I. 10 | 180 | 180, 78, 136, 51, 73, 106, 52, 152 | - |
| 63 | 7,85 | 1,02 | 1,00 | N.I. 11 | 184 | 73, 184, 77, 134, 59, 86, 69, 100, 174 | - |
| 44 | 8,40 | 1,68 | 1,30 | N.I. 4 | 131 | 73, 131, 147, 102, 133, 59, 132, 292 | - |
| 47 | 11,01 | 0,07 | 1,13 | N.I. 7 | 233 | 73, 147, 59, 133, 103, 304, 163, 233 | - |
| 73 | 11,12 | 1,69 | 1,31 | N.I. 15 | 273 | 73, 147, 59, 89, 103, 133, 215, 117, 273 | - |
| 64 | 11,45 | 1,42 | 1,28 | N.I. 12 | 246 | 73, 129, 147, 103, 155, 117, 133, 149 | - |
| 65 | 15,39 | 0,16 | 1,10 | N.I. 13 | 290 | 73, 103, 290, 232, 144, 262, 116, 291 | - |

Two additional samples were analyzed for comparison, a sample of *Aspergillus oryzae* feed additive obtained by conventional means (Amaferm®) and a sample which had been obtained following the fermenting procedure described above but dried by lyophilization on 5% silicon dioxide. The Amaferm® sample was manufactured using convetional operating procedures, that is, air-dried using a belt dryer on 50% wt/wt wheat bran. Altogether, the samples for the comparative analysis were:

- AO product obtained as detailed above dried on silicon dioxide at 40% wt/wt (AO40%) (pulse combustion dried or PCD)
- Amaferm® air dried on wheat bran (air dried)
- Lyophilized AO (freeze dried)

A metabolite Heatmap analysis [VIP>1.0, p-value<0.05] was prepared to visualize the differences among the samples. It was observed that the metabolite profile for AO40% obtained according to the method disclosed herein closely resembled that of the freeze-dried sample and substantially differed from Amaferm®. Freeze drying is considered a mild drying technique that greatly maintains the properties of the ingredients to be dried. Unfortunately, freeze drying AO at large scale presents several technical challenges difficult to overcome, requires large capital expenditure, and yields high operating costs (energy use and maintenance), so that it is not suitable for industrial production of AO-based biomass or compositions for animal feeding. In contrast, PCD used for drying AO40% is an energy-efficient, low-cost operating system proven to deliver top-quality dried products at industrial scale. Generally speaking, Amaferm® had a different metabolite profile when compared to the other samples. Many bioactive metabolites that were present in AO40% were either not found or present only in minor amounts in Amaferm®. Most notably, these included succinic acid, mevalonolactone, sebacic acid, Isopropylmalic acid, adonitol, glycolic acid, eyrthrono-1,4 lactone, glyceric acid, and kojic acid.

The metabolite profiling demonstrated that fermentation and drying procedure highly affect metabolite production by *Aspergillus oryzae.* The fermentation process disclosed herein optimizes bioactive metabolite production by the fungus, while PCD maintains their properties, such that the product obtained is better suited for food and feed supplementation.

**Example 2. Growth, survival and metabolic responses of whiteleg shrimp, *Litopenaeus vannamei*, fed different doses of *Aspergillus* oryzae biomass composition in commercial-style diets as a function of cold stress**

**Methods**

Experimental diets

A total of four experimental diets were formulated according to current industry standards and were produced by pelleting with a Cissonius pellet mill (3 Kw, 380 V, die Ø 120 mm, hole Ø 2,5 mm). The feed formulation is listed in Table 2. Before pelleting, all ingredients of each experimental diet were mixed thoroughly, sprayed with distilled water to reach a moisture content of approximately 10 % and was conditioned for 20 min at 45 °C. The temperature during the pelleting process did not exceed 65 °C. The freshly produced pellets were then dried at room temperature for a period of 48 h.

Table 2. Ingredients of the four experimental diets.

| Ingredient [g kg$^{-1}$] | Experimental Diet[1] | | | |
| | AOL (0.0125%) | AOM (0.01875%) | AOH (0.125%) | Control |
| --- | --- | --- | --- | --- |
| Fishmeal | 340 | 340 | 340 | 340 |
| Shrimp head meal | 90 | 90 | 90 | 90 |
| Soybean meal | 145 | 145 | 145 | 145 |
| Wheat meal | 367.875 | 367.8125 | 366.75 | 368 |
| Fish oil | 20 | 20 | 20 | 20 |
| Lecithin-Soy (70%) | 25 | 25 | 25 | 25 |
| Cholesterol | 2 | 2 | 2 | 2 |
| Vitamin & Mineral Premix | 5 | 5 | 5 | 5 |
| Gluten | 2.5 | 2.5 | 2.5 | 2.5 |
| Alginate | 2.5 | 2.5 | 2.5 | 2.5 |
| *Aspergillus oryzae* biomass composition[2] | 0.125 | 0.1875 | 1.25 | 0 |

[1]AOL = low dose of Aspergillus oryzae (AO) composition; AOM = medium dose of AO; AOL = high dose of AO.[2]Obtained as described in example 1

Feeding trial

The feeding experiments were made in a RAS shelving system comprising 16 separated 100 liter tanks, all supplied with the same artificial low salinity seawater (20 ppt). Aeration and water inflow was kept similar in all tanks. The photoperiod was from 7:30 - 20:00, following the lighting of the facility. Daily water parameter measurements included dissolved oxygen, temperature, conductivity and pH. Water samples were taken twice a week and analyzed for total ammonia-nitrogen, nitrite-nitrogen, and nitrate-nitrogen (Table 3).

Table 3. Water parameters during the course of the feeding experiment (mean $\pm$ SD).

| Water parameter | $O_2$ [%] | °C | mS/cm | pH | $NH_{4+}$ | $NO_{2-}$ | $NO_{3-}$ |
| --- | --- | --- | --- | --- | --- | --- | --- |
| Mean $\pm$ SD | 82.4 $\pm$ 10.8 | 28.1 $\pm$ 1.6 | 25.8 $\pm$ 1.3 | 7.7 $\pm$ 0.2 | 0.11 $\pm$ 0.04 | 0.19 $\pm$ 0.08 | 144.20 $\pm$ 32.93 |

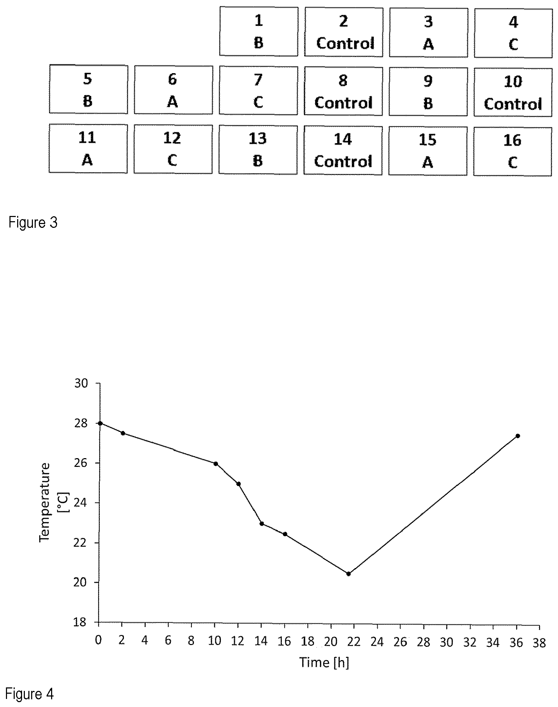

Each tank was stocked with 25 shrimps with an average bodyweight ranging from 2.05 g to 2.20 g. Any shrimp that died within the first 3 days of the trial was replaced with an average sized shrimp. The experimental diets were semi-randomly allocated to the tanks so that each experimental diet was represented in each shelving compartment of the RAS, illustrated in figure 3. The shrimps were fed two times a day at 9:00 and 15:00. The initial feeding rate was set at 15 % of the biomass and adjusted upwards thereafter in order to maintain an uneaten feed recovery of approximately 15 % after each feeding event. One hour after each feeding event (10:00 and 16:00) the uneaten feed remains were removed from the tanks via a suction hose into a separated bucket. To separate the feed remains from the feces, a swivel was created with a fine meshed net which made the lighter particles (feces) rise in the water column and the heavier particles (uneaten feed) remain on the bottom of the bucket. The separated feces were discarded and the uneaten feed remains were then transferred into aluminum cups and oven-dried overnight at 105 °C to constant weight, which was then recorded. The starting point of the uneaten feed recovery was shifted one tank to the right after each day (starting at 1) so that on average the time of the feeds in the tanks remained comparable. The carapaces of the molts found in each tank were recorded and counted as one molt unit.

Performance parameters of shrimp fed the experimental diets (Table 3) were defined as follows:

$$Mean\ individual\ weight\ gain = BWfinal - BWinitial$$

$$Molting\ frequency = \frac{total\ carapaces\ found}{days}$$

with:

Bwinitial=average initial bodyweight [g]
Bwfinal= average final bodyweight [g]

Total feed intake, feed conversion ratio, pellet stability and moisture

To determine the pellet moisture, 5 g of feed was dried at 105 °C for 24 h. Before weighing, samples were left to cool in a desiccator. Pellet moisture of the feeds was determined in triplicate. Pellet stability was estimated by the % of dry matter loss. To test stability 5 g of the feeds were placed in the same tanks they were allocated to with the same water and aeration conditions and were recovered after 1 h. The feeds were recovered in the same manner as during the feeding experiment and were dried over night at 105 °C and reweighed the following day. The total feed intake (total FI) and the feed conversion ratio (FCR) was calculated as follows:

$$Total\ FI = Feed\ input - pellet\ moisture - recovered\ uneaten\ feed - dry\ matter\ loss$$

$$FCR = \frac{Total\ FI}{Weight\ Gain}$$

whereas for calculating the feed conversion ratio, the weight gain refers to the biomass gain in each tank. Only replicates with survival rate greater than 60% were used for the calculation of FCR.

Cold stress induction

After week five of the feeding trial the shrimps were exposed to a cold stress shock. Water temperature was reduced to 20.5 °C over a period of 22 hours and was restored to the normal experiment condition approximately at the same gradual rate as shown in figure 4. The trial then continued for 1 week of feeding post stress.

**Results**

Table 4. Performance data of *L. vannamei* fed experimental diets (mean ± SD).

| Parameter | Experimental Diets | | | |
|---|---|---|---|---|
| | AOL | AOM | AOH | Control |
| Initial BW[1] [g] | 2.12 ± 0.38 | 2.16 ± 0.40 | 2.16 ± 0.42 | 2.15 ± 0.41 |
| Final BW [g] | 5.74 ± 1.76 | 6.91 ± 2.33 | 5.32 ± 1.54 | 6.04 ± 1.65 |
| Mean WG[2] [g] | 3.62 ± 0.59 | 4.75 ± 0.47 | 3.16 ± 0.49 | 3.89 ± 0.22 |
| MF[3] [moults/day] | 0.47 ± 0.11 | 0.57 ± 0.16 | 0.63 ± 0.16 | 0.61 ± 0.14 |
| Mortality [%] | 53.00 ± 11.02 | 37.00 ± 6.83 | 37.00 ± 14.38 | 46.00 ± 5.16 |
| Post Stress Mortality [Individual] | 2.75 ± 3.10 | 2.25 ± 1.5 | 2.25 ± 2.06 | 3.00 ± 0.82 |
| Total FI [g] | 180.38 ± 7.98 | 229.73 ± 23.85 | 210.65 ± 17.63 | 194.93 ± 26.17 |
| FCR [g$_{fed}$/g$_{gain}$] | 5.02 ± N/A | 3.81 ± 0.62 | 6.86 ± 4.40 | 5.08 ± N/A |

[1]BW= body weights; [2]WG= weight gain; [3]MF= moulting frequency.

Results are shown on table 4. The mortality throughout the experiment ranged from 24 to 64%. Mean mortality was lowest in diet treatment AOM and AOH, averaging a 20% reduction relative to the control. Post stress mortalities were 8% (AOL) to 25% (AOM and AOH) lower in all *Aspergillus oryzae* treatments when compared with the contr. The best growth was seen in shrimp receiving diet AOM with an average individual weight gain of 4.75 g, a 22% improvement compared with the control. The highest calculated total feed intake was observed for AOM, with a total feed intake of about 230 g during the course of the feeding trial. The most efficient feed conversion was seen in diet AOM with an FCR 25% better than the one attained by the control. The molting behavior was similar throughout all diet treatments with mean values ranging from 0.47 to 0.61 molts day$^{-1}$.

Altogether, the best overall performance (survival, growth) was observed in shrimps fed diet AOM (containing 0.01875 % *Aspergillus oryzae* biomass composition), followed by the control and diet AOH (containing 0.0125 % *Aspergillus oryzae* biomass composition). Post-stress mortalities were higher in control shrimps.

Further experiments showed that the *Aspergillus oryzae* biomass composition as contemplated in the present disclosure has a protective effect against white spot syndrome virus infection in shrimps (results showed a 20% mortality reduction when shrimp diet was supplemented with 0.2 g/kg of the *Aspergillus oryzae* biomass composition).

**Example 3. Influence of the inclusion of *Aspergillus* oryzae biomass composition on productive performance and egg quality produced by hens from 15 to 43 weeks of age.**

The object of this experiments was to study the influence of the inclusion of two *Aspergillus oryzae* (AO) products [AO postbiotic on silicon dioxide carrier (AOSO, which is the product obtained according to the present disclosure) at 50 g/ton of feed, and AO prebiotic on wheat bran carrier (AOWB, which is the feed additive obtained by conventional fermentation and drying, sold under the tradename Amaferm®) at 500 g/ton of feed] on productive performance and quality of the eggs of brown laying hens from 15 to 43 weeks of age.

**3.1. MATERIAL AND METHODS**

**Husbandry, Diets, and Experiment Design**

The procedures described in this research were approved by the Animal Ethics Committee of Universidad Politécnica de Madrid. In total, 72 Lohmann Brown Classic pullets, 15 wk of age, were selected at random from a commercial flock and housed in an environmentally controlled barn at the experimental facilities. The pullets were allotted individually to 72 enriched cages (70 cm x 50 cm x 62 cm) and fed a common commercial diet based on corn, wheat, and soybean meal for 28 wks. The cages were provided with an open trough feeder and 2 low pressure nipple drinkers. The lighting program started with 10 h/d of light and then, light was increased gradually to reach 16 h/d at 22 wks of age. Then, the light program was maintained constant throughout the experiment. Room temperature was recorded daily during the experiment and varied between 18 and 22 °C. Feed in mash form and water were provided for ad libitum consumption.

Treatments consisted of a control diet with 16.9% of CP and 2,750 kcal AMEn/kg and the same diet with either 50 g/ton of AOSO or 500 g/ton of AOWB. All diets were supplemented with a commercial enzyme complex with xylanase and β-glucanase activity. Also, titanium dioxide ($TiO_2$) was included at 0.5% in all diets as an inert marker to help in the determination of metabolizable energy. The experiment was completely randomized with 3 diets. Each treatment was replicated 24 times and the experimental unit was the cage with an individual pullet for all measurements.

Table 5. Ingredient composition and physic-chemical determination of the experimental diet (% as fed)

| Ingredient composition | |
|---|---|
| Corn | 39.50 |
| Wheat | 20.57 |
| Soybean meal, 47% CP | 13.08 |
| Sunflower meal, 35% CP | 11.55 |
| Calcium carbonate[1] | 9.73 |
| Soybean oil | 3.00 |
| Monocalcium phosphate | 0.63 |
| Mineral-vitamin premix[2] | 0.57 |
| Titanium dioxide | 0.50 |
| Sodium chloride 98% | 0.35 |
| DL-Met, 88% | 0.25 |
| L-Lys HCl, 78% | 0.23 |
| L-Thr, 99% | 0.04 |
| **Determined analysis** | |
| Moisture | 7.9 |
| Gross energy, Kcal/ kg | 3,655 |
| Ether extract | 5.7 |
| Crude protein | 16.0 |
| Total ash | 14.4 |
| Crude fiber | 4.5 |
| Neutral detergent fiber | 14.0 |
| **Calculated analysis** | |
| AMEn, Kcal/Kg | 2,750 |
| Ether extract | 5.26 |
| Crude fiber | 4.28 |
| Calcium | 3.90 |
| Phosphorus | 0.52 |
| Digestible phosphorus | 0.30 |
| Sodium | 0.14 |
| Chloride | 0.25 |
| **Digestible amino acids** | |
| Lys | 0.76 |
| Met | 0.45 |
| Met+Cys | 0.67 |
| Thr | 0.53 |
| Trp | 0.16 |
| Ile | 0.56 |
| Val | 0.64 |
| Arg | 0.94 |

[1]65% Coarse (2 to 4 mm) and 35% fine (<0.6 mm)

[2]Provided the following (per kilogram of diet): vitamin A (trans-retinyl acetate), 8,000 IU; vitamin D3 (cholecalciferol), 3,000 IU; vitamin E (dl-$\alpha$-tocopheryl acetate), 10 IU; vitamin K, 1 mg; vitamin B1, 1.0 mg; vitamin B2, 4.0 mg; vitamin B6, 1.5 mg; vitamin B12 (cyanocobalamin), 10 $\mu$g; niacin, 20 mg; pantothenic acid (d-calcium pantothenate), 8.2 mg; folic acid, 1.0mg; biotin, 100 $\mu$g; choline (choline chloride), 200 mg; manganese (MnO), 70 mg; zinc (ZnO), 50 mg; iron ($FeSO_4.H_2O$), 30 mg; copper ($CuSO_4$ $5H_2O$), 6 mg; iodine [$Ca(IO_3)_2$], 0.5 mg; selenium ($Na_2SeO_3$), 0.3 mg; Roxazyme, 200 mg [1,600 IU of endo-1,4-$\beta$-glucanase (EC 3.2.1.4), 3,600 IU of endo-1,3 (4)-$\beta$-glucanase (EC 3.2.1.6), and 5,200 IU of endo-1,4-$\beta$-xylanase (EC 3.2.1.8)] supplied by DSM S.A., Madrid, Spain; Axtra® PHY, 30mg [300 U of 4ª24 6-phytase (EC 3.1.3.26)] supplied by DuPont, Madrid, Spain.

**Measurements**

1.- *Hen Production.* Rate of egg production and egg weight were determined daily. Feed disappearance was determined by cage weekly. BW of the hens was measured by period (28 d). From these data, ADFI, egg production, egg weight, egg mass, feed conversion ratio (FCR) per kilogram of eggs, and BW gain of the hens were determined by week and for the entire experiment (15 to 43 wk of age). Any mortality was recorded and weighed as produced.

2.- *Egg Quality.* Non-saleable eggs (broken and shell-less eggs) were recorded by replicate daily. Other egg quality traits, including eggshell weight, strength, and thickness, were measured in all fresh eggs produced the last 2 d of each experimental period (28 d). Shell thickness was measured by triplicate at the middle section of the eggshell with a digital micrometer (model IT-014UT, Mitotuyo, Kawasaki, Japan). The average of the 3 measurements of each of the eggs was used for further analyses (Safaa, H. M., M. P. Serrano, D. G. Valencia, M. Frikha, E. Jiménez-Moreno, and G. G. Mateos. 2008a. Productive performance and egg quality of brown egg-laying hens in the late phase of production as influenced by level and source of calcium in the diet. Poult. Sci. 87:2043-2051). Shell strength, expressed in $g/cm^2$, was evaluated applying increased pressure to the broad pole of the egg using a press meter (Egg Force Reader, SANOVO Technology A/S, Odense, Denmark) as indicated by Safaa et al. (Safaa, H. M., M. P. Serrano, D. G. Valencia, X. Arbe, E. Jiménez-Moreno, R. Lázaro, and G. G. Mateos. 2008b. Effects of the levels of methionine, linoleic acid, and added fat in the diet of productive performance and egg quality of brown laying hens in the late phase of production. Poult. Sci. 87:1595-1602).

3.- *AME Determination.* Apparent metabolizable energy (AME )of the experimental diets were determined at 41 wk of age following accepted procedures (Gonzalez-Alvarado, J. M., E. Jiménez-Moreno, D. Gonzalez-Sanchez, R. Lázaro, and G. G. Mateos. 2010. Effect of inclusion of oat hulls and sugar beet pulp in the diet on productive performance and digestive traits of broilers from 1 to 42 days of age. Anim. Feed Sci. Technol. 162:37-46.). The equation used for determination was:

$$AME\ (Kcal\ per\ kg\ of\ diet) = GEdiet - [GE\ excreta * (\frac{TiO2\ diet}{TiO2\ excreta})]$$

where GE = gross energy Kcal per kg of sample (diet or excreta) and $TiO_2$ = concentration of titanium dioxide (diet or excreta).

**3.2. RESULTS**

The effects of the inclusion of *Aspergillus oryzae* products in the diet on productive performance of the hens from 15 to 43 wk of age are shown in Tables 6. Overall, hens fed AO products containing diets had increased egg production, improved FCR, and reduced shell-less eggs than the control hens. Compared to the control group, feeding AOSO improved egg produciton (1.1%), egg weight (0,5%), egg mass (1,2%), BW gain (4,4%), FCR (2,3%), and shell quality (2,2% less broken eggs and 56,2% less shell-less eggs). Compared to the grorup fed AOWB, feeding AOSO increased egg weight (1,2%), egg mass (1,6%), and BW gain (7,4%), while reduced the percentage of shell-less eggs by 15,4%.

Table 6. Effects of the inclusion of two products based on *Aspergillus oryzae* (AOSO and AOWB) on productive performance and shell quality of laying hens from 15 to 43 wk of age

| | Control | AOSO | AOWB | SEM | Relative change[1], % | |
| --- | --- | --- | --- | --- | --- | --- |
| | - | 50 ppm | 500 ppm | n=24 | 1 | 2 |
| Egg production, % | 81.7 | 82.6 | 82.4 | 0.711 | 1,1 | 0,2 |
| Feed intake, g/d | 105 | 104 | 102 | 1.30 | -1.0 | 1,9 |
| Egg weight, g | 59.1 | 59.4 | 58.7 | 0.803 | 0,5 | 1,2 |
| Egg mass, g/d | 48.7 | 49.3 | 48.5 | 0.647 | 1,2 | 1,6 |
| FCR, kg/kg | 2.18 | 2.13 | 2.13 | 0.030 | -2,3 | 0,0 |
| BW gain, g | 505 | 527 | 488 | 23.5 | 4,4 | 7,4 |
| Shell quality | | | | | | |

(continued)

| | Control | AOSO | AOWB | SEM | Relative change[1], % | |
|---|---|---|---|---|---|---|
| | - | 50 ppm | 500 ppm | n=24 | 1 | 2 |
| Broken eggs, % | 0.43 | 0.42 | 0.25 | 0.152 | -2,3 | 40,5 |
| Shell-less eggs, % | 0.89 | 0.39 | 0.45 | 0.195 | -56,2 | -15,4 |

1[(AOSO - Control)/Control)]*100; 2= [(AOSO - AOWB)/AOWB)]*100

The effects on egg quality of the inclusion of the *Aspergillus oryzae* products in the diet from 22 to 43 weeks of age are shown in Table 7. Overall, hens fed AO products containing diets had improved egg quality traits (lesss broken and shell-less eggs, stronger shells). Compared to control hens, feeding AOSO reduced the percentage of broken and shell-less eggs by 14,3 and 62,5%, respectively. This was associated with an increase in shell strength (2,5%) and a higher proportion of larger eggs (2,4% more L eggs, 68,4% more XL eggs, and 21,5% less S eggs). Compared to AOWB-fed hens, feeding AOSO reduced the proportion of shell-less eggs (44,4%) while improved egg grading by reducing the percentage of S eggs by 72,1% and increasing the contribution of L eggs by 14,7%.

Table 7. Effects of the inclusion of two products based on *Aspergillus oryzae* (AOSO and AOWB) on egg quality from 22 to 43 wk of age

| | Control | AOSO | AOWB | SEM | Relative change[1] | |
|---|---|---|---|---|---|---|
| | - | 50 ppm | 500 ppm | n=24 | 1 | 2 |
| **Egg quality traits** | | | | | | |
| Broken eggs, % | 0.35 | 0.30 | 0.18 | 0.167 | -14,3 | 40,0 |
| Shell-less eggs, % | 0.72 | 0.27 | 0.39 | 0.182 | -62,5 | -44,4 |
| Shell strength, g/cm$^2$ | 5.13 | 5.26 | 5.30 | 0.114 | 2,5 | -0,8 |
| Shell thickness, $\mu$m | 402 | 401 | 402 | 3.43 | -0,2 | -0,2 |
| **Egg size, %Total eggs** | | | | | | |
| S (< 53 g) | 8.14 | 6.39 | 11.0 | 2.79 | -21,5 | -72,1 |
| M (53 g to 63 g) | 62.6 | 63.2 | 62.9 | 5.10 | 1,0 | 0,5 |
| L (63 g to 73 g) | 28.6 | 29.3 | 25.0 | 5.57 | 2,4 | 14,7 |
| XL (> 73 g) | 0.66 | 1.11 | 1.10 | 0.527 | 68,2 | 0,9 |

1[(AOSO - Control)/Control)]*100; 2= [(AOSO - AOWB)/AOWB)]*100

The effects of the inclusion of *Aspergillus oryzae* in the diet presented as AOSO or AOWB on AME content of the diet are shown in Table 8. The AME of the diets increased with AOSO supplementation by 1.4 and 0.8% compared with AOWB and control diet fed hens, respectively.

Table 8. Effects of the inclusion of two products based on *Aspergillus oryzae* (AOSO and AOWB) on apparent metabolizable energy (AME, Kcal/kg) of the diet in laying hens at 41 wk of age

| | Control | AOSO | AOWB | SEM | Relative change[1] | |
|---|---|---|---|---|---|---|
| | - | 50 ppm | 500 ppm | n=24 | 1 | 2 |
| AME (Kcal/kg) | 2,679 | 2,701 | 2,665 | 23.2 | 0.8 | 1.4 |

1[(AOSO - Control)/Control)]*100; 2= [(AOSO - AOWB)/AOWB)]*100

Further experiments showed that the *Aspergillus oryzae* biomass composition as contemplated in the present disclosure improved productive performance and lifespan of laying hens in a commercial operation of egg production (results showed increased egg production by 1%, egg mass by 1%, L-graded eggs by 4% along with a 29% reduction in mortality when the diet was supplemented with 50 g/ton of the *Aspergillus oryzae* biomass composition from 24 to 41 weeks of age).

**Claims**

1. A process for preparing a composition comprising biomass from a non-pathogenic *Aspergillus* and bioactive compounds, the process comprising culturing the non-pathogenic *Aspergillus* in a liquid medium comprising an assimilable carbon source and inorganic nutrients, wherein the culturing comprises:

   (i) a first fermentation under aerobic conditions, and
   (ii) a second fermentation under stress conditions comprising hypoxic stress and pH stress conditions.

2. The process according to the preceding claim, wherein the inorganic nutrients are selected from the list consisting of K, Mg, S, Fe, Zn, Co, Cu, Na, Mo, Ca, Cl, and combinations thereof.

3. The process according to the preceding claim, wherein the assimilable carbon source of the liquid medium is selected from the group consisting of sucrose, glucose, beet pulp, starch, maltose, maltodextrin, glucose, fructose, galactose, lactose, and combinations thereof.

4. The process according to any one of the preceding claims, wherein:

   - the fermentation under aerobic conditions (i) is allowed to proceed until the pH of the culture is in the range from 4.0 to 6.0,
   - the culture is adjusted to pH from 8 to 12 before the fermentation under stress conditions (ii), and

   the fermentation under stress conditions (ii) is allowed to proceed until the pH of the culture is in the range from 4.0 to 7.5.

5. The process according to any one of the preceding claims, further comprising conditioning the composition by mixing with a carrier material, in particular, mixing with a carrier material selected from the group consisting of silicon dioxide, magnesium sulfate, calcium carbonate, calcium citrate, calcium phosphate, sodium chloride, potassium chloride, clays, dextrins, dextrose, maltodrextrins, bran, and combinations thereof.

6. The process according to claim 5, further comprising a step (iii) of drying.

7. The process according to the preceding claim, wherein the drying is performed by pulsed combustion drying.

8. The process according to claim 7, wherein the target moisture content after the drying step (iv) is less than 5% w/w.

9. A composition comprising biomass from a non-pathogenic *Aspergillus* and bioactive compounds obtainable by the process as defined in any one of the preceding claims.

10. The composition according to claim 9, wherein the bioactive compounds comprise alpha-linked heteroligosaccharides comprising galactose and mannose residues, mevalonolactone, isopropylmalic acid, and adonitol.

11. The composition according to claim 10, further comprising at least one bioactive compound selected from the group consisting of succinic acid, sebacic acid, glycolic acid, eyrthrono-1,4 lactone, glyceric acid, kojic acid, sebacic acid, lysophosphatidylethanolamine 18:2, lysophosphatidylcholine 18:1, 9,10,13 TriHOME, 9,12,13 TriHOME, 12,13 Di-HOME, azelaic acid, and combinations thereof.

12. A food or feed supplement comprising a composition as defined in any one of claims 9-11.

13. A food or feed comprising a composition as defined in any one of claims 9-11 or a supplement as defined in claim 12.

14. Use of a composition as defined in in any one of claims 9-11 as a postbiotic or as a prebiotic.

15. The process according to any one of claims 1-8, the composition according to any one of claims 9-11, the food or feed supplement according to claim 12, the food or feed according to claim 13, or the use according to claims 14, wherein the non-pathogenic *Aspergillus* is *Aspergillus oryzae,* in particular, *Aspergillus oryzae* NRRL 458.

Figure 1

| SD40% | SD60% | Air-dried | | | | | Freeze-dried | | | | | Tentative Identifications | |
|---|---|---|---|---|---|---|---|---|---|---|---|---|---|
| | | 0 h | B-C | | F-H | | 0 h | B-C | | F-H | | | |
| | | | 72-96 h | 144-168 h | 72-96 h | 144-168 h | | 72-96 h | 144-168 h | 72-96 h | 144-168 h | | |
| 0.23 | 0.44 | 0.01 | 0.01 | 0.01 | 0.01 | 0.01 | 0.04 | 2.51 | 3.56 | 3.60 | 3.59 | Propanoic acid derivatives 1 (1) | Organic acids |
| 0.35 | 3.07 | 0.26 | 0.49 | 0.26 | 0.33 | 0.54 | 1.07 | 0.73 | 1.00 | 0.93 | 1.31 | Lactic acid (2) | |
| 1.72 | 3.46 | 0.08 | 0.07 | 0.07 | 0.07 | 0.09 | 1.34 | 0.14 | 0.17 | 0.12 | 0.19 | Glycolic acid (3) | |
| 0.38 | 0.97 | 1.64 | 2.20 | 2.19 | 2.05 | 2.12 | 0.24 | 0.33 | 0.31 | 0.27 | 0.40 | Pyruvic acid (4) | |
| 1.43 | 1.27 | 0.04 | 0.16 | 0.13 | 0.18 | 0.24 | 0.06 | 1.84 | 1.84 | 1.82 | 1.91 | Succinic acid (5) | |
| 1.77 | 0.65 | 0.00 | 0.00 | 0.00 | 0.00 | 0.03 | 0.03 | 1.24 | 2.62 | 1.30 | 3.51 | Mevalonic lactone (6) | |
| 0.30 | 0.13 | 1.50 | 2.37 | 2.90 | 4.20 | 2.61 | 0.13 | 0.04 | 0.02 | 0.08 | 0.02 | Malic acid (7) | |
| 0.15 | 1.56 | 0.16 | 0.48 | 0.47 | 0.63 | 0.48 | 1.13 | 0.98 | 2.30 | 1.63 | 2.70 | α-Ketoglutaric acid (8) | |
| 4.65 | 0.18 | 0.21 | 0.39 | 0.36 | 0.61 | 0.55 | 0.03 | 0.00 | 0.01 | 0.01 | 0.02 | Citric acid (9) | |
| 0.37 | 5.92 | 0.02 | 0.01 | 0.01 | 0.01 | 0.01 | 0.02 | 0.03 | 0.03 | 0.03 | 0.03 | Glyceraldehyde (10) | carbohydrates |
| 3.06 | 1.46 | 1.28 | 0.16 | 0.13 | 0.17 | 0.27 | 0.71 | 0.14 | 0.21 | 0.12 | 0.24 | Glyceric acid (11) | |
| 1.64 | 4.28 | 0.00 | 0.00 | 0.00 | 0.00 | 0.00 | 0.22 | 0.06 | 0.13 | 0.07 | 0.15 | Erythrono-1,4-lactone (12) | |
| 0.39 | 5.86 | 0.00 | 0.00 | 0.00 | 0.00 | 0.01 | 0.01 | 0.04 | 0.08 | 0.04 | 0.09 | Carbohydrate 1 (13) | |
| 0.38 | 5.09 | 0.12 | 0.18 | 0.22 | 0.29 | 0.20 | 0.79 | 0.05 | 0.09 | 0.05 | 0.09 | Carbohydrate 2 (14) | |
| 1.20 | 1.04 | 0.02 | 0.17 | 0.17 | 0.17 | 0.21 | 0.01 | 2.08 | 2.33 | 1.75 | 2.49 | Erythritol (15) | |
| 1.11 | 1.13 | 0.08 | 0.21 | 0.17 | 0.20 | 0.14 | 0.58 | 2.59 | 1.84 | 1.50 | 2.10 | Carbohydrate 3 (16) | |
| 3.66 | 1.82 | 0.11 | 0.13 | 0.11 | 0.11 | 0.14 | 0.10 | 0.08 | 0.07 | 0.06 | 0.07 | Sugar acids derivatives (17) | |
| 0.49 | 4.29 | 1.11 | 0.06 | 0.05 | 0.08 | 0.06 | 0.04 | 0.67 | 0.61 | 0.36 | 0.65 | Ribose (18) | |
| 5.33 | 0.32 | 0.02 | 0.00 | 0.00 | 0.00 | 0.00 | 0.01 | 0.01 | 0.01 | 0.00 | 0.02 | Adonitol (19) | |
| 1.85 | 2.99 | 0.12 | 0.05 | 0.05 | 0.04 | 0.04 | 0.08 | 0.78 | 0.61 | 0.74 | 0.57 | Carbohydrate 4 (20) | |
| 4.47 | 0.45 | 0.39 | 0.19 | 0.25 | 0.29 | 0.38 | 0.04 | 0.09 | 0.18 | 0.13 | 0.10 | Carbohydrate 5 (21) | |
| 4.46 | 0.48 | 0.40 | 0.20 | 0.22 | 0.27 | 0.36 | 0.04 | 0.05 | 0.22 | 0.13 | 0.12 | Carbohydrate 6 (22) | |
| 0.31 | 1.44 | 0.72 | 0.49 | 0.50 | 0.71 | 0.63 | 0.00 | 2.18 | 2.24 | 1.11 | 2.26 | Sorbitol (23) | |
| 0.88 | 1.79 | 0.00 | 0.00 | 0.00 | 0.00 | 0.07 | 0.29 | 2.05 | 2.21 | 1.67 | 2.19 | Carbohydrate 7 (24) | |
| 0.48 | 1.05 | 1.54 | 1.27 | 1.10 | 1.26 | 1.03 | 0.12 | 1.43 | 1.25 | 0.99 | 1.28 | Carbohydrate 8 (25) | |
| 0.75 | 1.99 | 0.56 | 0.13 | 0.24 | 0.26 | 0.29 | 0.20 | 1.70 | 1.76 | 1.45 | 1.77 | Carbohydrate 9 (26) | |
| 1.29 | 1.25 | 0.21 | 0.16 | 0.15 | 0.19 | 0.14 | 0.19 | 2.01 | 1.94 | 2.02 | 1.65 | Carbohydrate 10 (27) | |
| 3.38 | 0.63 | 0.02 | 0.04 | 0.04 | 0.03 | 0.07 | 1.57 | 0.72 | 0.70 | 0.67 | 0.67 | Carbohydrate 11 (28) | |
| 0.02 | 0.00 | 1.00 | 2.70 | 2.72 | 2.73 | 2.76 | 2.99 | 0.00 | 0.00 | 0.00 | 0.00 | Sucrose (29) | |
| 3.33 | 0.34 | 0.18 | 0.35 | 0.48 | 0.47 | 0.60 | 2.40 | 0.15 | 0.22 | 0.31 | 0.17 | Lactose (30) | |
| 4.32 | 0.41 | 0.19 | 0.19 | 0.22 | 0.32 | 0.25 | 0.26 | 0.19 | 0.28 | 0.39 | 0.21 | Lactose (31) | |
| 4.85 | 0.56 | 0.00 | 0.00 | 0.00 | 0.00 | 0.00 | 0.00 | 0.11 | 0.22 | 0.25 | 0.15 | Carbohydrate 12 (32) | |
| 4.18 | 0.42 | 0.20 | 0.33 | 0.35 | 0.41 | 0.42 | 0.29 | 0.14 | 0.25 | 0.29 | 0.20 | Maltose (33) | |
| 0.17 | 0.17 | 2.37 | 2.81 | 3.13 | 2.15 | 2.96 | 0.22 | 0.10 | 0.17 | 0.10 | 0.14 | Palmitic acid (34) | Fatty acids and lipids |
| 0.15 | 0.09 | 1.63 | 3.30 | 3.59 | 2.39 | 3.16 | 0.06 | 0.02 | 0.10 | 0.05 | 0.10 | Linoleic acid (35) | |
| 0.15 | 0.08 | 2.41 | 3.19 | 3.47 | 2.16 | 2.96 | 0.06 | 0.02 | 0.06 | 0.03 | 0.06 | Elaidic acid (36) | |
| 0.29 | 0.40 | 1.95 | 2.37 | 2.49 | 1.73 | 2.34 | 0.86 | 0.32 | 0.46 | 0.36 | 0.44 | Stearic acid (37) | |
| 0.23 | 0.05 | 2.69 | 2.63 | 3.16 | 2.88 | 2.67 | 0.19 | 0.01 | 0.01 | 0.01 | 0.01 | Monoolein (38) | |
| 0.14 | 0.25 | 3.61 | 2.25 | 2.16 | 2.64 | 2.45 | 0.54 | 0.11 | 0.09 | 0.09 | 0.09 | 5-oxo-proline (39) | others |
| 5.05 | 0.46 | 0.00 | 0.00 | 0.00 | 0.00 | 0.00 | 0.25 | 0.05 | 0.06 | 0.06 | 0.05 | Kojic acid (40) | |
| 0.58 | 0.78 | 1.45 | 1.60 | 1.59 | 1.56 | 1.74 | 1.14 | 0.68 | 0.63 | 0.54 | 0.67 | N.I. 1 (41) | unknowns |
| 0.84 | 0.90 | 1.22 | 1.27 | 1.23 | 1.28 | 1.18 | 1.30 | 0.77 | 0.83 | 0.79 | 0.81 | N.I. 2 (42) | |
| 0.66 | 0.80 | 1.35 | 1.57 | 1.59 | 1.57 | 1.54 | 1.50 | 0.59 | 0.53 | 0.61 | 0.52 | N.I. 3 (43) | |
| 5.34 | 0.06 | 0.02 | 0.02 | 0.02 | 0.02 | 0.03 | 0.03 | 0.10 | 0.09 | 0.04 | 0.26 | N.I. 4 (44) | |
| 0.53 | 0.13 | 0.86 | 1.84 | 2.02 | 1.98 | 1.61 | 4.43 | 0.18 | 0.15 | 0.12 | 0.10 | N.I. 5 (45) | |
| 5.05 | 0.46 | 0.00 | 0.00 | 0.00 | 0.00 | 0.00 | 0.25 | 0.05 | 0.06 | 0.06 | 0.05 | N.I. 6 (46) | |
| 1.99 | 0.59 | 0.39 | 0.35 | 0.32 | 0.34 | 0.31 | 0.08 | 0.90 | 1.75 | 1.96 | 1.93 | N.I. 7 (47) | |
| 0.00 | 0.00 | 2.97 | 3.16 | 2.55 | 4.00 | 2.32 | 0.00 | 0.00 | 0.00 | 0.00 | 0.00 | N.I. 8 (48) | |

Figure 2

| | | 1<br>B | 2<br>Control | 3<br>A | 4<br>C |
|---|---|---|---|---|---|
| 5<br>B | 6<br>A | 7<br>C | 8<br>Control | 9<br>B | 10<br>Control |
| 11<br>A | 12<br>C | 13<br>B | 14<br>Control | 15<br>A | 16<br>C |

Figure 3

Figure 4

# EP 4 371 416 A1

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BAMPIDIS VASILEIOS ET AL: "Safety of the fermentation product of Aspergillus oryzae NRRL 458 (Amaferm ) as a feed additive for dairy cows (Biozyme Inc.)", THE EFSA JOURNAL, [Online] vol. 20, no. 2, 10 November 2021 (2021-11-10), XP093037840, Parma, IT ISSN: 1831-4732, DOI: 10.2903/j.efsa.2022.6983 Retrieved from the Internet: URL:https://api.wiley.com/onlinelibrary/tdm/v1/articles/10.2903/j.efsa.2022.6983> [retrieved on 2023-04-06] * Abstract * | 9,12,13, 15 | INV. A23K10/10 A23K10/16 A23K20/126 A23K20/142 A23K20/158 A23K20/163 A23K30/20 A23K50/75 A23K50/80 A23L21/00 A23K10/30 |
| X | CN 115 316 491 A (SHANDONG TAISHAN SHENGLIYUAN GROUP CO LTD) 11 November 2022 (2022-11-11) * page 6, paragraph 006 * | 9 | |
| X | Zhu Jinlong ET AL: "EVALUATION OF ASPERGILLUS ORYZAE POSTBIOTIC ON NUTRIENT DIGESTIBILITY, GUT MICROBIOME, AND METABOLOME IN GROWING PIGS FED HIGH FIBER DIETS", A DISSERTATION SUBMITTED TO THE FACULTY OF UNIVERSITY OF MINNESOTA BY Jinlong Zhu IN PARTIAL FULFILLMENT OF THE REQUIREMENTS FOR THE DEGREE OF DOCTOR OF PHILOSOPHY, 31 March 2022 (2022-03-31), XP093037841, Retrieved from the Internet: URL:https://conservancy.umn.edu/bitstream/handle/11299/241351/Zhu_umn_0130E_23205.pdf?sequence=1 [retrieved on 2023-04-06] * table 1-8 * | 9,12-15 | TECHNICAL FIELDS SEARCHED (IPC) A23K C11C A23L |

-/--

The present search report has been drawn up for all claims

| Place of search | Date of completion of the search | Examiner |
|---|---|---|
| Munich | 21 April 2023 | Steiner Ribeiro G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 22 38 3118

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 5 025177 B2 (IDEMITSU KOSAN CO) 12 September 2012 (2012-09-12) * paragraphs [0009] – [0011] * ----- | 9,11-14 | |
| X | US 2018/035704 A1 (WICKING J BRUCE [US] ET AL) 8 February 2018 (2018-02-08) | 9-15 | |
| Y | * lines 59-60 – column 1 * * line 65 – column 1, line 73 * ----- | 1-8 | |
| Y | US 2022/312797 A1 (PIPAN MIHA [GB]) 6 October 2022 (2022-10-06) * paragraph [0138]; example 5 * ----- | 1-8 | |
| A | Anonymous: "Amaferm", , 31 January 2021 (2021-01-31), pages 1-2, XP093037839, Retrieved from the Internet: URL:https://madbarn.com/wp-content/uploads /2021/11/Amaferm-BioZyme-Guaranteed-Analys is.pdf [retrieved on 2023-04-05] * the whole document * ----- | 1-15 | TECHNICAL FIELDS SEARCHED (IPC) |

The present search report has been drawn up for all claims

| Place of search | Date of completion of the search | Examiner |
|---|---|---|
| Munich | 21 April 2023 | Steiner Ribeiro G. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 22 38 3118

This annex lists the patent family members relating to the patent documents cited in the above-mentioned European search report.
The members are as contained in the European Patent Office EDP file on
The European Patent Office is in no way liable for these particulars which are merely given for the purpose of information.

21-04-2023

| Patent document cited in search report | | Publication date | Patent family member(s) | | Publication date |
|---|---|---|---|---|---|
| CN 115316491 | A | 11-11-2022 | NONE | | |
| JP 5025177 | B2 | 12-09-2012 | JP 5025177 | B2 | 12-09-2012 |
| | | | JP 2007325580 | A | 20-12-2007 |
| US 2018035704 | A1 | 08-02-2018 | AU 2016224128 | A1 | 07-09-2017 |
| | | | BR 112017018166 | A2 | 10-04-2018 |
| | | | CA 2977287 | A1 | 01-09-2016 |
| | | | CN 107427053 | A | 01-12-2017 |
| | | | EP 3261459 | A1 | 03-01-2018 |
| | | | JP 2018507709 | A | 22-03-2018 |
| | | | KR 20170118934 | A | 25-10-2017 |
| | | | US 2018035704 | A1 | 08-02-2018 |
| | | | WO 2016134406 | A1 | 01-09-2016 |
| US 2022312797 | A1 | 06-10-2022 | AU 2018248695 | A1 | 14-11-2019 |
| | | | CA 3058703 | A1 | 11-10-2018 |
| | | | CN 110494045 | A | 22-11-2019 |
| | | | EP 3606355 | A1 | 12-02-2020 |
| | | | GB 2561348 | A | 17-10-2018 |
| | | | US 2022312797 | A1 | 06-10-2022 |
| | | | WO 2018185474 | A1 | 11-10-2018 |
| | | | ZA 201906590 | B | 27-01-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SAFAA, H. M. ; M. P. SERRANO ; D. G. VALENCIA ; M. FRIKHA ; E. JIMÉNEZ-MORENO ; G. G. MATEOS.** Productive performance and egg quality of brown egg-laying hens in the late phase of production as influenced by level and source of calcium in the diet. *Poult. Sci.,* 2008, vol. 87, 2043-2051
- **SAFAA, H. M. ; M. P. SERRANO ; D. G. VALENCIA ; X. ARBE ; E. JIMÉNEZ-MORENO ; R. LÁZARO ; G. G. MATEOS.** Effects of the levels of methionine, linoleic acid, and added fat in the diet of productive performance and egg quality of brown laying hens in the late phase of production. *Poult. Sci.,* 2008, vol. 87, 1595-1602

- **GONZALEZ-ALVARADO, J. M. ; E. JIMÉNEZ-MORENO ; D. GONZALEZ-SANCHEZ ; R. LÁZARO ; G. G. MATEOS.** Effect of inclusion of oat hulls and sugar beet pulp in the diet on productive performance and digestive traits of broilers from 1 to 42 days of age. *Anim. Feed Sci. Technol.,* 2010, vol. 162, 37-46